Europäisches Patentamt

(19) European Patent Office

Office européen des brevets

(11) Numéro de publication: **0 169 259**
A1

## (12) DEMANDE DE BREVET EUROPEEN

(21) Numéro de dépôt: **84108785.1**

(22) Date de dépôt: **25.07.84**

(51) Int. Cl.⁴: **A 61 L 27/00**
//A61F2/06

(43) Date de publication de la demande:
**29.01.86 Bulletin 86/5**

(84) Etats contractants désignés:
**BE CH DE FR GB IT LI NL SE**

(71) Demandeur: **SURGICAL PATENT PRODUCTS INC. LTD.**
**c/o de la Guardia Arosemena & Benedetti P.O. Box 4150**
**Panama 5(PA)**

(72) Inventeur: **Mänz, Herbert**
**Am Vogelsberg**
**D-2430 Neustadt in Holstein(DE)**

(74) Mandataire: **Micheli, Michel-Pierre et al,**
**MICHELI & CIE 118, Rue du Rhône Case Postale 47**
**CH-1211 Genève 6(CH)**

(54) Prothèses vasculaires, pour conservation à sec leur procédé de conditionnement et leur application en chirurgie.

(57) La prothèse vasculaire souple, synthétique ou d'origine biologique est capable d'être conservée à sec. Elle contient au moins 10 % en poids d'eau et au moins 10 ‰ en poids d'un composé biocompatible, peu volatil, retenant l'eau, par rapport au poids de la prothèse sèche.

Selon le procédé de conditionnement de prothèses synthétiques ou d'origine biologique, pour conversation à sec, on soumet la prothèse à un trempage dans un bain aqueux où se trouve un composé biocompatible, peu volatil, à température ambiante, retenant l'eau de telle sorte qu' après séchage, elle contienne au moins 10 % en poids d'eau et 10 % en poids dudit composé par rapport au poids de la prothèse sèche.

Ces prothèses sont utilisées en chirurgie vasculaire ou cardiovasculaire.

EP 0 169 259 A1

Prothèses vasculaires, pour conservation à sec

leur procédé de conditionnement et

leur application en chirurgie

L'invention concerne des prothèses vasculaires à base de collagène de différentes origines biologiques qui peuvent être des homogreffes veineuses, hétérogreffes bovines, veines ombilicales traitées ou des prothèses vasculaires synthétiques à armature textile sur lesquelles est fixée une couche d'une protéine.

Les prothèses utilisées à l'heure actuelle sont conservées dans des tubes en verre au sein d'une solution bactéricide ou d'une solution à base d'alcool qui affecte toutefois leur biocompatibilité.

En effet, les tests "in vitro" plus précisément la méthode de culture organotypique à partir d'aortes d'embryons de poulets, ont été décevants. D'une façon générale, on peut dire que les milieux de conservation à base d'éthanol ou glutaraldéhyde ou d'autres bactéricides, malgré des rinçages répétés des prothèses avant utilisation, se sont montrés cytotoxiques in vi- vo.

Par ailleurs, si la conservation de telles prothèses dans du sérum physiologique leur confère une biocompatibilité convenable quant à la croissance cellulaire, il est vraisemblable que la cicatrisation ne peut s'accompagner d'une néoendothélialisation après l'implantation ; en effet, le voile cellulaire n'adhère que très pauvrement au support dans de tels cas.

Depuis 1968, on utilise des prothèses en fibres de polyester, sous forme de tissu gaufré, tissé ou tricoté, velouté à l'intérieur ou à l'extérieur, préparé en tubes ou plaques. Avant l'utilisation de telles
prothèses, il faut les précoaguler avec le sang du malade. La fibrine du sang rend les parois de la prothèse
imperméables au sang (empêche les fuites du sang dans les
tissus environnants), et crée une surface lisse à l'intérieur du vaisseau mais elle nécessite une neutralisation de la thrombine formée pendant la précoagulation.
La conservation de telles prothèses se fait "à sec"
dans des emballages stériles , mais leur préparation avant utilisation est longue et délicate.

Une amélioration de prothèses synthétiques a
résulté de leur imperméabilisation par une couche de diverses protéines telles que du collagène, des globulines,
de l'albumine, de la fibronectine, de la gélatine, de
l'élastine ou autres; toutefois, les protéines doivent
être solidement ancrées sur la trame textile, et il faut
immerger la prothèse textile dans une solution de la
protéine choisie et y fixer cette dernière par couplage
chimique par exemple par l'intermédiaire du glutaraldéhyde ou par du diphényl-carbodiimide. Ce procédé est
connu par l'homme de l'art.

On sait que les prothèses rendues ainsi étanches sont raides et cassantes et qu'elles ne peuvent pas
être conservées "à sec"; lorsqu'elles sont conservées en
milieu liquide, elles présentent le même inconvénient
que les greffons d'origine biologique : elles sont cytotoxiques.

La présente invention permet de remédier à cet
inconvénient ; en effet, elle permet la conservation
"à sec" des prothèses d'origine biologique ou des prothèses synthétiques, sous une forme souple, grâce à un
traitement conditionnant.

Le brevet français No 71.16.082 concerne un "procédé pour le traitement d'un fil à base de collagène, fil ainsi obtenu et ses applications", qui consiste à mettre en contact le fil avec au moins un bain d'eau distillée et d'agent de traitement capable de maintenir l'humidité du fil en empêchant l'évaporation de l'eau qui s'y trouve contenue.

De façon surprenante, on a trouvé qu'un traitement du même type mais adapté à la réalisation des prothèses, est applicable à la production de prothèses vasculaires en évitant ainsi les inconvénients inhérents à la technique antérieure et en supprimant la nécessité de conditionner les prothèses en présence d'un liquide.

Un premier objet de l'invention est donc une prothèse d'origine biologique ou une prothèse synthétique sur laquelle a été fixée une protéine, qui est souple et le reste, malgré une conservation "à sec", et contient au moins 10 % en poids d'eau et 10 % en poids par rapport au poids de la prothèse sèche d'un composé biocompatible, peu volatil, retenant l'eau. Ces prothèses peuvent être conservées après stérilisation, à l'abri des contaminations, sans être dans un milieu liquide, ce qui est un avantage de l'invention.

Un autre avantage de l'invention est le fait que le spécialiste n'aura besoin de faire subir aucun traitement préalable à la prothèse avant son implantation in vivo; il pourra l'utiliser telle que commercialisée, du fait de sa souplesse et de son absence de cytotoxicité.

Un tel résultat était tout à fait inattendu.

Un autre objet de l'invention est l'assouplissement et le conditionnement des prothèses vasculaires d'origine biologique ou synthétique par trempage dans une solution aqueuse contenant un composé biocompatible, peu volatil, retenant l'eau; ledit procédé de conditionnement peut être suivi d'une stérilisation habituelle par toute méthode connue en ce domaine.

L'invention concerne donc une prothèse contenant en permanence une quantité d'eau au moins égale à 10 % en poids par rapport au poids de la prothèse sèche et au moins un composé peu volatil, avide d'eau et pouvant notamment la retenir dans la prothèse en l'absence de tout milieu liquide, ledit composé étant présent à raison d'au moins 10 % et de préférence d'au moins 15 % en poids par rapport au poids de la prothèse sèche et ayant un poids moléculaire ne dépassant pas 400 environ.

Lesdits composés, agents de conditionnement notamment capables de maintenir l'eau dans la prothèse vasculaire peuvent être présents dans la couche superficielle et/ou dans la masse de la prothèse. Ils sont avides d'eau, plus ou moins hygroscopiques.

Leur présence, ainsi que celle de l'eau dans, ou sur la prothèse, lui donne une certaine souplesse qui se maintient au stockage.

Dans certains cas, on peut améliorer les propriétés de la prothèse en incorporant un produit biocompatible antiagrégant, anticoagulant, antiseptique, bactéricide ou similaire ou leurs mélanges dans le bain de conditionnement selon l'invention; lesdits produits seront absorbés par la prothèse conjointement à l'eau et à l'agent de conditionnement.

Les agents de conditionnement qui sont des corps organiques doivent être bien tolérés par les tissus du corps humain et donc être biocompatibles et aussi hémocompatibles.

De tels agents de conditionnement peuvent être choisis parmi les alcools et acides gras, leurs esters et éthers, les alcools et polyalcools et ayant un faible degré de volatilité à température ambiante, et leurs dérivés polycondensés tels que les polyoxyalkylène-glycols, de masse moléculaire inférieure à 400 environ et leurs mélanges. Parmi les composés préférés, on peut citer :
le glycérol, les glycols, le polyoxyéthylène-glycol.

La quantité d'agent de conditionnement varie évidemment avec la nature de ce dernier et avec le genre de la prothèse traitée. La quantité d'agent dans la prothèse est en général sensiblement égale à la quantité d'eau. L'homme de l'art peut aisément déterminer les proportions relatives optimales de l'eau et de l'agent de conditionnement particulier en procédant à des essais préliminaires.

On notera que la combinaison de l'eau avec un agent de conditionnement est indispensable pour l'obtention des résultats de l'invention. En effet, un agent de conditionnement même de nature hygroscopique, tel que le glycérol n'est pas capable d'être absorbé par les prothèses en l'absence d'eau.

Par ailleurs, l'eau, utilisée seule, est absorbée par la prothèse mais ne peut y rester de façon durable car elle s'évapore peu de temps après son application dès que la prothèse est sortie d'un milieu liquide. Selon l'invention, l'association eau-agent de conditionnement permet à la fois la pénétration de l'agent dans la prothèse et la rétention de l'eau qui y pénètre simultanément lors d'une conservation.

Le poids moléculaire de l'agent de conditionnement mélangé avec l'eau exerce une influence directe sur le gonflement de la prothèse; plus le poids moléculaire est élevé, moins la quantité de liquide absorbée est importante; d'autre part le gonflement n'est plus permanent si le poids moléculaire de l'agent est supérieur à 400.

Pour l'obtention d'une prothèse souple et pouvant être conservée hors d'un milieu liquide, conformément à l'invention, on peut opérer par exemple par trempage de la prothèse dans un bain constitué d'un mélange d'eau et de l'agent de traitement. Les agents de conditionnement peuvent être mis en oeuvre en solution, en émulsion ou en suspension aqueuse ou dans un tiers solvant.

La température à laquelle la prothèse est imprégnée du bain de conditionnement peut varier de 10 à 50°C et on peut s'écarter de cette gamme de température sans inconvénient dans la mesure où le bain reste liquide.

Le temps de traitement peut varier d'une seconde à plusieurs jours selon la nature de l'agent de conditionnement et sa concentration dans le bain. En général, des durées de 10 minutes à 8 heures conviennent. La durée de traitement dépend évidemment de facteurs tels que la température, la nature de la prothèse et du degré d'assouplissement désiré.

On ne sort pas du cadre de l'invention en réalisant le trempage des prothèses dans le bain de conditionnement à une pression supérieure à la pression atmosphérique ou même inférieure; on facilite alors le "débullage" ou dégazage.

Il est préférable de maintenir le pH du bain de conditionnement entre certaines limites pour obtenir après stérilisation une prothèse à pH voisin de la neutralité; un pH compris entre 5 et 9 convient.

Lorsque les prothèses conditionnées sont stérilisées par l'oxyde d'éthylène, le pH s'accroît légèrement au cours de la stérilisation. Il est préférable de compenser cette variation en introduisant dans le bain de conditionnement un acide faible, tel que l'acide acétique ou l'acide citrique, qui sera absorbé sur la prothèse;

La stérilisation peut également s'opérer par rayonnement et dans ce cas, aucune correction du pH n'est nécessaire.

Le bain de conditionnement peut contenir, outre des produits modificateurs de pH, des antiagrégants, des anticoagulants, des antiseptiques bactéricides ou similaires. Les prothèses absorberont ces produits lors du traitement d'assouplissement, selon l'invention, acquérant de nouvelles propriétés, ce qui est un autre intérêt de l'invention.

L'un des avantages de l'invention résulte de ce que la stérilisation des prothèses qui a lieu après leur conditionnement, est facilitée par la présence d'eau dans celles-ci; on sait, par exemple, que la stérilisation à l'oxyde d'éthylène ne convient que si l'article à stériliser contient une certaine humidité.

Après leur traitement d'assouplissement et leur stérilisation, les prothèses peuvent être conservées telles que, à sec, donc sans être dans un liquide mais seulement dans un emballage ne laissant pas passer de microorganismes. Il est ainsi possible d'utiliser des emballages en matière plastique, en papier traité ou en carton sous forme d'étui rigide ou thermoformé.

De plus, la prothèse peut être stérilisée dans son emballage, par action d'un gaz ou d'un rayonnement connu pour leur pouvoir stérilisant.

Le conditionnement des prothèses selon l'invention est avantageux pour leur fabricant mais aussi pour l'utilisateur.

La conservation stérile des prothèses traitées suivant le procédé n'est pas affectée par leur procédé de conditionnement et d'assouplissement mais dépend uniquement de la nature de la prothèse.

Toute manipulation pour enlever l'alcool ou un autre liquide de conservation des prothèses avant l'usage est supprimée. Les prothèses sont souples et faciles à utiliser.

Les prothèses selon l'invention ne sont pas cytotoxiques; au contraire in vivo, après implantation elles favorisent la croissance à leur surface de cellules de diverses origines et de différents types. Les implantations "in vivo" de matériaux traités suivant le procédé induisent une bonne affinité cellulaire et améliorent la colonisation cellulaire des prothèses.

L'invention sera maintenant illustrée, sans être aucunement limitée, par les exemples suivants :

EXEMPLE 1

On utilise un bain de conditionnement ayant la composition ci-après :

| Glycérol | 60 | parties en poids |
| Eau distillée | 40 | parties en poids |
| Acide citrique | 0,05 | parties en poids |

Une homogreffe est prélevée, préparée et rendue non antigénique par un procédé connu. Elle est ensuite calibrée et mise sur un tuteur pour qu'elle ne perde pas sa forme et mise à tremper dans le bain de conditionnement ci-dessus, à température ambiante.

Après immersion de 8 heures, elle est égouttée et, après quelques heures de séchage, elle est emballée dans un conditionnement en thermoformé puis stérilisée par action de l'oxyde d'éthylène ou de rayons ionisants.

L'utilisateur peut utiliser cette homogreffe telle qu'elle sort de son emballage, par exemple pour un pontage vasculaire.

On obtient des résultats évquivalents en traitant comme ci-dessus une hétérogreffe bovine ou une veine ombilicale. Ces prothèses peuvent être utilisées pour un pontage vasculaire.

EXEMPLE 2

Une prothèse tubulaire en polyester gaufré, tricoté, est trempée dans une solution tampon à pH 7,4 contenant 9 % d'albumine et 1, 5 % de glutaraldéhyde.

Après 15 minutes d'immersion, on laisse égoutter, sécher et on l'immerge pendant 3 heures à température ambiante dans un bain de conditionnement comme décrit à l'exemple 1.

La prothèse devient souple et reste souple à la conservation.

EXEMPLE 3

Une prothèse tubulaire en polyester gaufré, en tricot chaînette est trempée dans une solution tampon à pH 7,4 contenant 10 % d'un mélange de même poids de collagène et d'albumine. Après réticulation et séchage, la prothèse est assouplie par trempage pendant 2 h. 30 dans un bain de conditionnement tel que décrit dans l'exemple 1.

Après conditionnement et stérilisation, la prothèse peut être utilisée de suite ou stockée. Elle est souple, agréable à utiliser, ne nécessite pas de précoagulation, n'est pas cytotoxique et favorise la néoendothélialisation.

EXEMPLE 4 Bain de conditionnement

| | |
|---|---|
| Diéthylène glycol | 65 parties en poids |
| Eau distillée | 35 parties en poids |

EXEMPLE 5    Bain de conditionnement

Trishydroxyméthylpropare                    50 parties en poids

Eau distillée              50 parties en poids


EXEMPLE 6    Bain de conditionnement

Glycérol                   55 parties en poids

Eau distillée              45 parties en poids

Héparine                   10 000 unités


Les prothèses ainsi conditionnées ont des propriétés anticoagulantes localisées et temporaires.


EXEMPLE 7    Bain de conditionnement

Glycérol              60    parties en poids

Eau distillée         40    parties en poids

Acide acétylsalicylique               0,5 parties en poids


Les prothèses ainsi conditionnées ont des propriétés antiplaquettaires localisées et temporaires.

REVENDICATIONS

1. Prothèses vasculaires souples, synthétiques ou d'origine biologique, capables d'être conservées à sec, caractérisées en ce qu'elles contiennent au moins 10 % en poids d'eau et au moins 10 % en poids d'un composé biocompatible, peu volatil, retenant l'eau, par rapport au poids de la prothèse sèche.

2. Prothèses selon la revendication 1, caractérisées en ce que le composé retenant l'eau possède une masse moléculaire inférieure à 400 environ et est choisi parmi les alcools et acides gras, leurs esters et éthers, les alcools et polyalcools peu volatils à température ambiante et leurs dérivés de polycondensation tels que les polyoxyalkylènes, glycols et leurs mélanges.

3. Prothèses selon la revendication 2, caractérisées en ce qu'elles contiennent de 10 à 50 % en poids d'eau par rapport au poids de la prothèse sèche.

4. Prothèses selon l'une quelconque des revendications précédentes, caractérisées en ce que le composé retenant l'eau est un polyol choisi parmi le glycérol, le diéthylène-glycol et le tris(hydroxyméthyl)propane.

5. Prothèses selon l'une quelconque des revendications précédentes, caractérisées en ce que le composé retenant l'eau représente de 10 à 70 % en poids du poids de la prothèse sèche.

6. Prothèses selon l'une quelconque des revendications précédentes, caractérisées en ce que l'eau et le composé retenant l'eau sont localisés dans la masse de

la prothèse.

7.      Prothèses synthétiques à armatures en textile synthétique sur lesquelles a été fixée une couche de protéines par couplage chimique, caractérisées en ce que l'eau et le composé retenant l'eau sont localisées dans ladite couche.

8.      Prothèses selon l'une quelconque des revendications précédentes, caractérisées en ce qu'elles contiennent, en outre, des composés antiagrégants, anticoagulants, antiseptiques, bactéricides, ou antibiotiques, biocompatibles ou leurs mélanges.

9.      Procédé de conditionnement de prothèses synthétiques ou d'origine biologique, pour conservation à sec, caractérisé en ce qu'on soumet la prothèse à un trempage dans un bain aqueux où se trouve un composé biocompatible, peu volatil, à température ambiante, retenant l'eau de telle sorte qu'après séchage, elle contienne au moins 10 % en poids d'eau et 10 % en poids dudit composé par rapport au poids de la prothèse sèche.

10.      Procédé selon la revendication 9, caractérisé en ce que la prothèse, après conditionnement et séchage, est stérilisée par exposition à un gaz ou à un rayonnement.

11.      Procédé selon l'une des revendications 9 ou 10, caractérisé en ce que l'on ajoute au bain de conditionnement des produits antiagrégants ou anticoagulants ou antiseptiques ou bactéricides ou antibiotiques ou leurs mélanges.

0169259

12.      Application des prothèses selon l'une quelconque des revendications 1 à 8, en chirurgie vasculaire
ou cardiovasculaire.

**0169259**
Numéro de la demande

Office européen
des brevets

**RAPPORT DE RECHERCHE EUROPEENNE**

EP 84 10 8785

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 357 274 (H.W. HEINZ) <br><br> * Colonne 2, lignes 30-44; revendication 1 * | 1,3-6, 9 | A 61 L 27/00 // <br> A 61 F 2/06 |
| Y | | 2,7,8, 10-12 | |
| | --- | | |
| D,Y | FR-A-2 135 433 (D. VIVIEN) <br> * Page 5, lignes 4-10; exemples 4-9; revendications 1-19 * | 2,10 | |
| | --- | | |
| Y | GB-A-1 018 288 (SPOFA) <br><br> * Page 2, lignes 60-127; page 3, exemple; revendications 1,6,9 * | 7,8,11 ,12 | |
| | --- | | **DOMAINES TECHNIQUES RECHERCHES (Int. Cl.4)** |
| A | GB-A- 994 275 (M. GERENDAS) | | A 61 L 27/00 |
| | --- | | |
| A | DE-A-2 024 341 (INSTITUTUL PENTRU CONTROLUL DE STAT AL MEDICAMENTULUI SI CERCETARI FARMACEUTICE) | | |
| | ----- | | |

Le present rapport de recherche a été établi pour toutes les revendications

| Lieu de la recherche | Date d'achèvement de la recherche | Examinateur |
|---|---|---|
| LA HAYE | 19-03-1985 | PELTRE CHR. |